(19) Europäisches Patentamt / European Patent Office / Office européen des brevets

(11) **EP 4 502 155 A1**

(12) **EUROPEAN PATENT APPLICATION**
published in accordance with Art. 153(4) EPC

(43) Date of publication:
**05.02.2025 Bulletin 2025/06**

(51) International Patent Classification (IPC):
**C12N 11/096** (2020.01)   **C02F 3/34** (2023.01)

(21) Application number: **23779759.2**

(52) Cooperative Patent Classification (CPC):
**C02F 3/34; C12N 11/096;** Y02W 10/10

(22) Date of filing: **17.03.2023**

(86) International application number:
**PCT/JP2023/010639**

(87) International publication number:
**WO 2023/189743 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.03.2022 JP 2022059131**

(71) Applicant: **Mitsubishi Chemical Corporation Tokyo 100-8251 (JP)**

(72) Inventors:
• **GOTOU Hisanori**
  **Tokyo 100-8251 (JP)**
• **KAWAGISHI Tomoki**
  **Tokyo 100-8251 (JP)**

(74) Representative: **Hoffmann Eitle Patent- und Rechtsanwälte PartmbB Arabellastraße 30 81925 München (DE)**

(54) **MICROBIAL CARRIER AND WATER TREATMENT METHOD**

(57)    The present invention provides a microbial carrier in which denitrification efficiency is stabilized and improved, and a water treatment method using the same. The microbial carrier of the present invention is a microbial carrier which carries a microorganism having denitrification ability, the microbial carrier comprising an iron-containing carrier which comprises a biodegradable resin and iron, in which, when the iron-containing carrier comprises phosphorus, a molar ratio (phosphorus/iron) X of the phosphorus to the iron in the iron-containing carrier is $0 < X \leq 0.5$, and when the iron-containing carrier comprises no phosphorus, the molar ratio (phosphorus/iron) X is $X = 0$, and an iron content of the iron-containing carrier is $1.0 \times 10^{-4}$% by mass or more and less than 2.0% by mass with respect to a mass of the iron-containing carrier.

FIG. 1

EP 4 502 155 A1

**Description**

[Technical Field]

The present invention relates to a microbial carrier and a water treatment method.
Priority is claimed on Japanese Patent Application No. 2022-059131, filed March 31, 2022, the content of which is incorporated herein by reference.

[Background Art]

As a treatment method for water containing ammonia nitrogen, a method of using a two-stage reaction using microorganisms is known. The method consists of a nitrification reaction of converting ammonia into nitric acid using a microorganism having an ability to nitrify (hereinafter, may be referred to as "nitrifying bacterium") and a denitrification reaction of decomposing the nitric acid into nitrogen using a microorganism having an ability to denitrify (hereinafter, may be referred to as "denitrifying bacterium"), and since the generated nitrogen is released into the air, the method is an environmentally friendly denitrification method.
Since many of the denitrifying bacteria used in the denitrification reaction are heterotrophic bacteria, the denitrification reaction requires a carbon source. As a representative carbon source, methanol is known, but methanol has toxicity and is problematic in terms of work safety. In addition, in order to obtain stable denitrification ability, it is necessary to supply, to the water to be treated, methanol in an amount corresponding to a concentration of nitric acid nitrogen, but it is not easy to control the supply amount. Furthermore, for example, when denitrification treatment is performed on rearing water of an aquatic organism, there is a problem in that the methanol remaining without being consumed has an adverse effect on the aquatic organism.
As a method for solving such a problem, Patent Document 1 proposes a biological denitrification method in which, instead of methanol, a biodegradable resin is added to the water to be treated. A biodegradable resin is advantageous in that, since the carbon source has a sustained release property, the denitrification ability can be maintained without requiring precise control of the addition amount. However, denitrification efficiency such as the denitrification rate and amount of denitrification is not sufficient. In recent years, release standards of nitric acid nitrogen have been stricter from the viewpoint of environmental protection, and there is a demand for the development of a denitrification treatment method with a higher denitrification efficiency.
In a method of increasing the denitrification efficiency, the growth, activation, and the like of a denitrifying bacterium are considered important factors. It is known that a denitrifying bacterium is activated by a nutrient such as a phosphate, an activator such as a mineral, and the like. Patent Document 2 proposes a microbial carrier in which an activator such as a mineral is combined. However, details of an appropriate element type, concentration, and the like to improve the denitrification efficiency are still unknown.

[Citation List]

[Patent Documents]

[Patent Document 1]
Japanese Unexamined Patent Application, First Publication No. 2004-175848
[Patent Document 2]
Japanese Unexamined Patent Application, First Publication No. 2004-008923

[Summary of Invention]

[Technical Problem]

In the biological denitrification treatment using a microbial carrier (hereinafter, may be simply referred to as "carrier") comprising a biodegradable resin, the denitrifying bacterium is fixed on the surface of the carrier to form a biofilm. In addition, the carbon source required for the denitrification is mainly provided by the biodegradable resin constituting the carrier, but in this case, it is also necessary to provide with a nutrient such as a phosphate, an activator such as a mineral, and the like in addition to the carbon source. In particular, iron is an essential component for efficiently removing nitrogen in order to form the biofilm by efficiently fixing the denitrifying bacterium on the carrier.
Therefore, in the denitrification reaction, it is important that the denitrifying bacterium can satisfactorily take in iron. In order to provide the iron component, a method of adding and replenishing iron or an iron compound to the water to be treated in advance is considered, but at a pH in a neutral range or near-neutral range where the denitrifying bacterium can maintain the activity, the iron is rapidly oxidized by oxygen and exists as trivalent iron. Trivalent iron is mainly precipitated as insoluble iron hydroxide. Therefore, there is a problem in that the iron concentration in the water to be treated is likely to decrease, and the denitrifying bacterium cannot satisfactorily take in iron.

The present invention has been made in view of the above circumstances, and an object thereof is to provide a microbial carrier in which denitrification efficiency is stabilized and improved, and a water treatment method using the same.

[Solution to Problem]

As a result of intensive studies to achieve the above-described objects, the present inventors have found that, since iron which can be taken in by the denitrifying bacterium is only iron present in the vicinity of the surface of the carrier, an iron-containing carrier comprising iron in a biodegradable resin is effective in efficiently supplying iron to the denitrifying bacterium. Furthermore, in order to improve denitrification, suitable type of iron compound and suitable conditions of combination with phosphorus compound was found out.

The present invention has the following configurations.

[1] A microbial carrier which carries a microorganism having denitrification ability, the microbial carrier comprising:

an iron-containing carrier which comprises a biodegradable resin and iron,
in which, when the iron-containing carrier comprises phosphorus, a molar ratio (phosphorus/iron) X of the phosphorus to the iron in the iron-containing carrier is $0 < X \leq 0.5$,
when the iron-containing carrier comprises no phosphorus, the molar ratio (phosphorus/iron) X is X = 0, and
an iron content of the iron-containing carrier is $1.0 \times 10^{-4}\%$ by mass or more and less than 2.0% by mass with respect to a mass of the iron-containing carrier.

[2] The microbial carrier according to [1], further comprising:

a phosphorus-containing carrier which comprises a biodegradable resin and phosphorus,
in which, when the phosphorus-containing carrier comprises iron, a molar ratio (iron/phosphorus) Y of iron to the phosphorus in the phosphorus-containing carrier is $0 < Y \leq 0.1$, and
when the phosphorus-containing carrier comprises no iron, the molar ratio (iron/phosphorus) Y is Y = 0.

[3] The microbial carrier according to [1] or [2],
in which the iron-containing carrier comprises trivalent iron.
[4] The microbial carrier according to any one of [1] to [3],
in which the biodegradable resin is a biodegradable polyester.
[5] The microbial carrier according to [4],
in which the biodegradable polyester includes two or more types of constitutional units derived from a dicarboxylic acid.
[6] The microbial carrier according to any one of [1] to [5],
in which the microbial carrier is a microbial carrier which further carries a microorganism having an ability to decompose the biodegradable resin.
[7] A water treatment method using a microbial carrier which carries a microorganism having denitrification ability,

in which the microbial carrier comprises an iron-containing carrier comprising a biodegradable resin and iron,
when the iron-containing carrier comprises phosphorus, a molar ratio (phosphorus/iron) X of the phosphorus to the iron in the iron-containing carrier is $0 < X \leq 0.5$,
when the iron-containing carrier comprises no phosphorus, the molar ratio (phosphorus/iron) X is X = 0, and
an iron content of the iron-containing carrier is $1.0 \times 10^{-4}\%$ by mass or more and less than 2.0% by mass with respect to a mass of the iron-containing carrier.

[8] The water treatment method according to [7],

in which the microbial carrier further comprises a phosphorus-containing carrier comprising a biodegradable resin and phosphorus,
when the phosphorus-containing carrier comprises iron, a molar ratio (iron/phosphorus) Y of iron to the phosphorus in the phosphorus-containing carrier is $0 < Y \leq 0.1$, and

when the phosphorus-containing carrier comprises no iron, the molar ratio (iron/phosphorus) Y is $Y = 0$.

[Advantageous Effects of Invention]

According to the microbial carrier and the water treatment method of the present invention, the denitrification efficiency is stabilized and improved.

[Brief Description of Drawings]

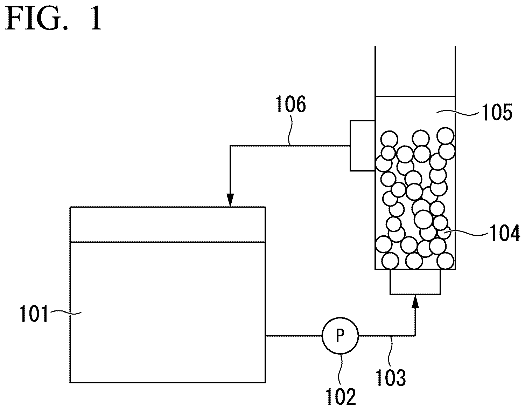

FIG. 1 is a schematic diagram showing an example of a denitrification treatment system.

[Description of Embodiments]

Hereinafter, embodiments of the present invention will be described in detail; however, the following description is only a representative example of the embodiments, and the present invention is not intended to be limited to these embodiments and can be carried out with various modifications to the extent of the gist of the invention.

Meanings of terms are as follows.

Denitrifying bacterium means a microorganism having an ability to denitrify.

Decomposing bacterium means a microorganism having an ability to decompose a biodegradable resin.

Microorganism means a microorganism including the denitrifying bacterium and the decomposing bacterium. The denitrifying bacterium and the decomposing bacterium may be the same species. That is, a microorganism which corresponds to both the denitrifying bacterium and the decomposing bacterium may be present.

Low-molecular-weight organic moiety means an organic moiety which has become lower in molecular weight by decomposition of the biodegradable resin by the microorganism.

Carrier A means an iron-containing carrier comprising a biodegradable resin and iron. Carrier A may further comprise phosphorus.

Carrier B means a phosphorus-containing carrier comprising a biodegradable resin and phosphorus. Carrier B may further comprise iron.

Microbial carrier means a carrier including one or more of carrier A. The microbial carrier may further include one or more of carrier B.

Total organic carbon (TOC) means the concentration of organic substances present in water. TOC is measured using a combustion-type TOC analyzer "TOC-300V" manufactured by Nittoseiko Analytech Co., Ltd.

Total iron means soluble and insoluble iron contained in water. Total iron is measured by an ICP light emission spectral analysis method described in JIS K0102 57.4.

Total phosphorus means soluble and insoluble phosphorus contained in water. Total phosphorus is measured by a molybdenum blue light absorption photometry method described in JIS K0102 46.3.1 to 3.

"to", indicating a numerical range, means that the numerical values described before and after "to" are included as the lower limit value and the upper limit value. The numerical value range described in the present specification can be any numerical value range of any combination of the lower limit value and the upper limit value thereof.

<Microbial carrier>

The microbial carrier according to the embodiment will be described in detail.

The microbial carrier is a microbial carrier which carries a microorganism having denitrification ability.

The microbial carrier includes at least one carrier A. The microbial carrier may include two or more carriers A. The microbial carrier may further include one or more carriers B, in addition to carrier A. When including two or more carriers, the microbial carrier is an aggregate of the carriers.

The microbial carrier is preferably a microbial carrier which carries a microorganism having an ability to decompose a biodegradable resin.

Iron is derived from an iron compound, and is preferably comprised in carrier A in the state of the iron compound. In addition, an iron compound having insolubility with respect to water in a neutral range of pH 6 to 8 is preferable, and as the type of iron compound, iron (III) oxide, iron (II, III) oxide, and iron (III) hydroxide, each of which includes trivalent iron, are exemplary examples.

With carrier A comprising these iron compounds, in water to be treated, the pH in the vicinity of the surface of carrier A is maintained in a pH range suitable for the microorganism fixed on the surface of the carrier to be active. Therefore, after the iron is replenished from carrier A, denitrification is improved. In addition, when the microorganism decomposes the biodegradable resin to remove nitrogen, the trivalent iron of carrier A is reduced to divalent iron by the action of the microorganism, and is taken in by the microorganism or is taken in by the microorganism in a trivalent iron state.

Therefore, since iron is not dissociated from carrier A other than being taken in by the microorganism and the surplus iron is not released and remains on the side of the water to be treated, it is possible to efficiently replenish the iron necessary for the denitrification by the microorganism.

In general, iron which can be taken in by a microorganism is mainly divalent iron. However, divalent iron makes the treatment water acidic by elution. In an acidic environment, divalent iron is oxidized to trivalent iron, so that microorganisms cannot efficiently take in the iron. In the case of trivalent iron, the treatment water exhibits neutrality, and the trivalent iron is reduced to divalent iron by the action of microorganisms, so that the microorganisms can efficiently take in the iron.

The iron content C(Fe) of carrier A is preferably $1.0 \times 10^{-4}$% by mass or more and less than 2.0% by mass, more preferably $2.0 \times 10^{-4}$% by mass or more and 1.8% by mass or less, still more preferably $5.0 \times 10^{-4}$% by mass or more and 1.5% by mass or less, and particularly preferably $1.5 \times 10^{-3}$% by mass or more and $8.0 \times 10^{-1}$% by mass or less with respect to the mass of carrier A. The decomposing bacterium decomposes and consumes the biodegradable resin on the surface of carrier A into the low-molecular-weight organic moiety, and at the same time, consumes dissolved oxygen in the vicinity of the surface, thereby inducing an anoxic environment in the vicinity of the surface of carrier A. Thereafter, the denitrifying bacterium performs the denitrification by taking in and using the iron of carrier A. When the iron content of carrier A is within the above-described range, the decomposing bacterium efficiently produces and consumes the low-molecular-weight organic moiety, and the anoxic environment is formed favorably, so that the denitrification is rapidly and stably performed. When the iron content of carrier A is $1.0 \times 10^{-4}$% by mass or more, the iron is sufficiently replenished by carrier A, and the denitrification efficiency is improved. When the iron content of carrier A is less than 2.0% by mass, the low-molecular-weight organic moiety is consumed by the decomposing bacterium, and at the same time, an anoxic environment is formed, so that denitrification is stabilized and denitrification efficiency is improved. When the iron content is 2.0% by mass or more, it is presumed that the aggregation of the biofilm, in which iron ions are eluted from carrier A in the process of forming the biofilm on the surface of carrier A, causes a decrease in denitrification.

In addition, in order for the denitrifying bacterium to become fixed on carrier A and efficiently consume the low-molecular-weight organic moiety, phosphorus is also an important nutrient source. When the microbial carrier comprises phosphorus, it can efficiently provide phosphorus to denitrifying bacteria.

When carrier A comprises phosphorus, the molar ratio (phosphorus/iron) X of phosphorus to iron, comprised in carrier A, is more than 0 and 0.5 or less ($0 < X \leq 0.5$), preferably 0.1 or less and more preferably 0.05 or less. When the molar ratio X of phosphorus/iron in carrier A is within the above-described range, since iron and phosphorus are efficiently replenished to the microorganism, denitrification is improved.

When the molar ratio X of phosphorus/iron in carrier A is a predetermined value or more, a compound which is difficult to incorporate for microorganism, such as iron phosphate (III) may be generated. Since carrier A comprises more iron than phosphorus, it is presumed that, in an anaerobic environment in which the activity of the denitrifying bacterium is active, some of the trivalent iron is reduced to divalent iron by the microorganism, and some of the iron phosphate (III) is also incorporated into the microorganism.

When carrier A does not comprise phosphorus, the molar ratio (phosphorus/iron) X of the phosphorus to the iron is 0 (X = 0).

In addition, when carrier B comprising the biodegradable resin and phosphorus is used in combination with carrier A, it is expected that the denitrification efficiency can be improved by replenishing with iron and phosphorus. A mixing ratio when combining carrier A and carrier B is preferably a mass ratio of carrier A/ carrier B of 10/90 or more. Within this range, iron and phosphorus are efficiently replenished to the microorganism, so that the microorganism is reliably fixed on carrier A and carrier B, and the denitrification efficiency is improved.

The phosphorus content in carrier B is not particularly limited, but is preferably 0.002% to 0.5% by mass and more preferably 0.005% to 0.1% by mass with respect to the weight of carrier B. When the phosphorus content is within the above-described range, the phosphorus is efficiently replenished to the microorganism. Therefore, the microorganism is reliably fixed on the microbial carrier, and the denitrification efficiency is improved.

When carrier B comprises iron, the molar ratio (iron/phosphorus) Y of iron to phosphorus, comprised in carrier B, is 0 to 0.1 ($0 < Y \leq 0.1$), preferably 0.05 or less. When the molar ratio Y of iron/phosphorus in carrier B is within the above-described range, since the iron and phosphorus are efficiently replenished to the microorganism, the denitrification efficiency is improved.

When carrier B does not comprise iron, the molar ratio (iron/phosphorus) Y of the iron to the phosphorus is 0 (Y = 0).

Carrier B comprises more phosphorus than iron, and carrier B replenishes the microorganism with phosphorus in any of an aerobic environment, an anaerobic environment, or an anoxic environment, but in an aerobic environment, trivalent irons are not reduced and it is difficult for iron phosphate (III) to be incorporated into the organism. Therefore, in an anaerobic environment or an anoxic environment, in which carrier A is easily replenished with iron, iron phosphate (III) is relatively easily incorporated into the microorganism. On the other hand, carrier B can replenish phosphorus in an aerobic environment, an anaerobic environment, or an anoxic environment, and in an aerobic environment, iron phosphate (III) is less likely to be taken in by the microorganism. Therefore, it is preferable that the upper limit of the molar ratio X of phosphorus to iron in carrier A be larger than the upper limit of the molar ratio Y of iron to phosphorus in carrier B.

Phosphorus comprised in carrier A and carrier B is not particularly limited, and for example, a calcium compound such as calcium phosphate and a magnesium compound such as magnesium phosphate are exemplary examples.

The iron content C(Fe) (% by mass) and the phosphorus content C(P) (% by mass) of carrier A and carrier B are measured by the following (I) to (VII).

(I) 10 g of carrier A or carrier B and 30 ml of a 2 mol/l sodium hydroxide aqueous solution are weighed in a pressure-resistant tube having a capacity of 50 to 100 ml, and the tube is tightly closed.

(II) The mixture is heated at 105°C for 12 hours, and then cooled to around 20°C using tap water or the like.

(III) After the cooling, the entire amount of the aqueous solution $\alpha$ present in the pressure-resistant tube is taken out and transferred to a 200 ml glass beaker.

(IV) The pH of the aqueous solution $\alpha$ is adjusted to 7.0 using 2 mol/l of hydrochloric acid.

(V) The aqueous solution $\alpha$ in which the pH is adjusted is transferred to a 100 ml volumetric flask, and pure water is further added thereto to adjust the volume of the aqueous solution $\alpha$ to 100 ml.

(VI) The TOC $\alpha$ (mg/l), the total iron $\alpha$ (mg/l), and the total phosphorus $\alpha$ (mg/l) of the aqueous solution $\alpha$ are measured by the above-described measuring method.

(VII) The iron content C(Fe) is obtained by the following expression 1; and the phosphorus content C(P) is obtained by the following expression 2.

$$C(Fe) \text{ (\% by mass)} = \text{Total iron } \alpha \text{ (mg/l)} \div \text{TOC } \alpha \text{ (mg/l)} \times 100 \text{ ... Expression 1}$$

$$C(P) \text{ (\% by mass)} = \text{Total phosphorus } \alpha \text{ (mg/l)} \div \text{TOC } \alpha \text{ (mg/l)} \times 100$$

Expression 2

The iron comprised in carrier A is preferably an iron compound having insolubility in water having a pH of 6 to 8. When carrier A comprises an iron compound having solubility in water in a neutral range of pH 6 to 8, in a case where carrier A is immersed in the water, the iron compound having solubility is rapidly eluted from carrier A into the water, and the pH of carrier A in the vicinity of the surface of carrier A is lowered to less than 5. Therefore, carrier A is significantly reduced in the fixation of the microorganism and the activity of the microorganism.

Shapes of carrier A and carrier B are not particularly limited, and may be any shape such as a cylindrical shape, a spherical shape, and a chip shape. From the viewpoint of ease of filling a denitrification tank, it is preferable that carrier A and carrier B have the shape of a cylinder, a sphere, or the like.

The microbial carrier according to the embodiment of the present invention comprises a biodegradable resin. A low-molecular-weight organic component generated by hydrolysis of the biodegradable resin is used as a carbon source required for the denitrification.

The biodegradable resin is preferably a biodegradable polyester. As the biodegradable polyester, for example, a polylactic acid (PLA)-based compound, a polybutylene succinate (PBS)-based compound, a poly caprolactone (PCL)-based compound, a poly hydroxybutyrate (PHB)-based compound such as poly-3-hydroxybutyrate-co-3-hydroxyhexanoate (PHBH) and poly-3-hydroxybutyrate-co-3-hydroxyvalerate (PHBV), a polyhydroxy alkanoate (PHA)-based compound, a poly-3-hydroxyrate (P3HA)-based compound, a polybutylene adipate/terephthalate (PBAT), poly(terephthalate/succinate), and the like are exemplary examples.

Among the biodegradable polyester, a biodegradable polyester having a constitutional unit derived from a dicarboxylic acid is preferable, and a biodegradable polyester having a constitutional unit derived from a dicarboxylic acid and a constitutional unit derived from a diol is more preferable. When the biodegradable resin is a biodegradable polyester, the iron comprised in carrier A can be more efficiently incorporated into the body by the denitrifying bacterium, so that the proliferation and activity of the denitrifying bacterium are stable, and the denitrification efficiency is improved.

As the dicarboxylic acid, for example, oxalic acid, malonic acid, succinic acid, adipic acid, glutaric acid, suberic acid, azelaic acid, sebacic acid, terephthalic acid, isophthalic acid, phthalic acid, and the like are exemplary examples. The biodegradable resin preferably has two or more kinds of constitutional units derived from the dicarboxylic acid. When a biodegradable resin having two or more kinds of constitutional units derived from the dicarboxylic acid is used, a denitrification rate is higher than that when a biodegradable resin having one kind of constitutional unit derived from a dicarboxylic acid is used, and a tendency to exhibit higher denitrification performance is obtained.

As the biodegradable resin, a biodegradable resin having a constitutional unit derived from succinic acid among the above-described dicarboxylic acids is preferable. A biodegradable resin mainly having a butylene succinate unit, such as polybutylene succinate (PBS), is preferable. As a suitable PBS-based biodegradable resin, for example, polybutylene succinate, poly(butylene succinate/adipate) (PBSA), poly(butylene succinate/carbonate), poly(butylene adipate/terephthalate) (PBAT), and poly(ethylene terephthalate/succinate) are exemplary examples. Among these, particularly from the viewpoint of high biodegradability and the viewpoint that a carbon source required for the denitrification can be slowly sustained, PBSA is preferable. Furthermore, since the PBSA is more easily decomposed than other biodegradable resins such as the PHB-based resin, the generation of a low-molecular-weight organic component is improved. At the same time, since dissolved oxygen is also consumed at the time of generating the low-molecular-weight organic component, the vicinity of the biodegradable resin is in an anoxic state, and an anoxic environment effective for the denitrifying bacterium to perform the denitrification reaction is formed. In addition, the low-molecular-weight organic component derived from the PBSA is preferably a substrate or a hydrogen donor for the denitrifying bacterium to grow and propagate.

The biodegradable resin may be a mixture of a biodegradable polyester having a constitutional unit derived from a carboxylic acid, and a resin such as polylactic acid, PHA, polyvinyl alcohol, and cellulose. By mixing the biodegradable resin having a constitutional unit derived from a dicarboxylic acid with these resins having different biodegradabilities, the biodegradable resin can be used as a carbon source for a long period of time.

The microbial carrier according to the embodiment of the present invention may further comprise a resin other than a biodegradable resin, within a range not impairing the effect of the present invention. As other resins, for example, polyvinyl alcohol, polyethylene, polypropylene, polyethylene terephthalate, polybutylene terephthalate, polyacetic acid, polyvinyl chloride, polystyrene, and the like are exemplary examples.

A method for producing carrier A is not particularly limited, and for example, a method in which iron and/or an iron compound is kneaded and dispersed in advance in a high concentration in the biodegradable resin to prepare a masterbatch, and then the masterbatch and the biodegradable resin are supplied to a uniaxial or biaxial kneading and extruding machine or the like to be melted is an exemplary example.

A method for producing carrier B is not particularly limited. For example, a method in which phosphorus and a phosphorus compound are kneaded and dispersed in advance in a high concentration in the biodegradable resin to prepare a masterbatch, and then the masterbatch and the biodegradable resin are supplied to a uniaxial or biaxial kneading and extruding machine or the like to be melted is an exemplary example.

The denitrifying bacterium carried by the microbial carrier according to the embodiment of the present invention is not particularly limited. A known bacterium having denitrification ability can be appropriately selected and used, but a heterotrophic denitrifying bacterium is preferable.

In addition, the microbial carrier according to the embodiment of the present invention preferably carries a microorganism having a decomposition ability of the biodegradable resin (hereinafter, may be simply referred to as "decomposing bacterium"). The decomposing bacterium adheres to the microbial carrier and decomposes the biodegradable resin comprised in the microbial carrier. As a result, more carbon sources are supplied to the denitrifying bacterium than in a case in which the decomposing bacterium is not carried by the microbial carrier, and the denitrifying bacterium is promoted to grow or to be active. As a result, the denitrification rate and the denitrification amount are improved. The decomposing bacterium is not particularly limited, and known bacteria having the ability to decompose a biodegradable resin can be appropriately used.

The water to be treated, which is a target in the purification treatment using the microbial carrier, is not particularly limited as long as it contains nitrous acid nitrogen and/or nitric acid nitrogen. The water to be treated may be the rearing water of the aquatic organism described above, or may be drainage such as livestock drainage, food drainage, sewage, and purified tank drainage. In addition, the water to be treated may be fresh water or sea water.

"Sea water" means water containing salt at a concentration of 3.2% by mass or more.

"Brackish water" means water containing salt at a concentration of 0.05% by mass or more and less than 3.2% by mass.

On the other hand, "fresh water" may be water containing no salt at all, or may be water containing salt at a concentration lower than the salinity concentration of brackish water.

A nitric acid nitrogen concentration of the water to be treated is not particularly limited, but is preferably 10 mg-N/L or more, more preferably 20 mg-N/L or more, still more preferably 50 mg-N/L or more, and particularly preferably 100 mg-N/L or more; and is preferably 5,000 mg-N/L or less and more preferably 2,000 mg-N/L or less. When the nitric acid nitrogen concentration of the water to be treated is within the above-described range, the denitrification proceeds efficiently.

The iron concentration of the water to be treated is not particularly limited, but is preferably 0 mg-Fe/L or more, more preferably 0.01 mg-Fe/L or more, still more preferably 0.1 mg-Fe/L or more, and particularly preferably 0.5 mg-Fe/L or more; and is preferably 1 mg-Fe/L or less. When the iron concentration of the water to be treated is within the above-described range, the denitrification proceeds efficiently.

A phosphorus concentration of the water to be treated is not particularly limited, but is preferably 0.01 mg-P/L or more, more preferably 0.1 mg-P/L or more, still more preferably 1.0 mg-P/L or more, and particularly preferably 5.0 mg-P/L or more; and is preferably 20 mg-P/L or less and more preferably 10 mg-P/L or less. When the phosphorus concentration of the water to be treated is within the above-described range, the denitrification proceeds efficiently.

<Water treatment method>

The water treatment method according to the embodiment will be described in detail.

The water treatment method relates to a denitrification treatment method using the microbial carrier according to the embodiment described above. In the water treatment method, the microbial carrier including one or more carriers A is used as a microbial carrier carrying a microorganism having denitrification ability.

More specifically, the water treatment method includes a denitrification treatment step of supplying the water to be treated to a denitrification tank filled with the microbial carrier carrying the denitrifying bacterium, and performing denitrification by converting nitrous acid nitrogen and/or nitric acid nitrogen in the water to be treated into nitrogen by a denitrification reaction.

(Denitrification treatment step)

In the water treatment method, a method of supplying the water to be treated to the denitrification tank filled with the microbial carrier is not particularly limited. For example, a method of supplying the water to be treated to the denitrification tank using a denitrification treatment system of a circulation type, an overflow type, an intermittent type, or a batch type is an exemplary example. Among these, a circulation-type denitrification treatment system is preferably used.

FIG. 1 is a schematic diagram showing an example of the denitrification treatment system. The denitrification treatment system shown in FIG. 1 includes a water storage tank 101, a pump 102, a supply tube 103, a denitrification column 105 filled with a microbial carrier 104, and a return tube 106. In the circulation-type denitrification treatment system, the water to be treated, put into the water storage tank 101, is circulated by being supplied to the denitrification column 105 through the supply tube 103 by the pump 102, coming into contact with the microbial carrier 104, and then being returned to the water storage tank 101 through the return tube 106.

In the denitrification treatment step, the temperature of the water to be treated is not particularly limited, but is preferably 0°C or higher, more preferably 10°C or higher and still more preferably 20°C or higher; and preferably 40°C or lower, more preferably 35°C or lower and still more preferably 30°C or lower. The water to be treated may contain nitric acid nitrogen, and may contain ammonia nitrogen and nitrous acid nitrogen. In addition, the water to be treated may also contain an organic component derived from other than the biodegradable resin. As suitable examples of the water to be treated, rearing water used for aquaculture of aquatic organisms is an exemplary example. The water treatment method is suitably used for carrying out the denitrification treatment of rearing water after nitrification treatment containing nitric acid nitrogen, in which the microorganism is carried and the activity of the denitrifying bacterium is stabilized and promoted.

The circulation-type denitrification treatment system may include various other elements not shown in FIG. 1. As such elements, for example, a transfer unit for transferring the water to be treated such that the water is supplied to the denitrification tank, such as a siphon; a heater and/or a cooler for adjusting the temperature of the water to a temperature suitable for the denitrification reaction; a nitrifying carrier carrying nitrifying bacterium which converts ammonia nitrogen into nitric acid when the water contains ammonia nitrogen; and the like are exemplary examples.

The water treatment method may be incorporated into a water treatment method using a two-stage reaction of a nitrification reaction of converting ammonia into nitric acid and a denitrification reaction of decomposing the nitric acid into nitrogen. As the water treatment method, for example, a method of purifying rearing water of aquatic organisms such as salmon, trout, sweetfish, char, eel, crab, and shrimp is an exemplary example. In this case, since the rearing water contains ammonia nitrogen, it may be supplied to a nitrification tank provided with a nitrification carrier carrying the nitrifying bacterium to convert the ammoniacal nitrogen into nitrous acid nitrogen and/or nitric acid nitrogen, and the rearing water containing nitrous acid nitrogen and/or nitric acid nitrogen, which passes through the nitrification tank, (the water to be treated applicable to the water treatment method) may be supplied to the denitrification treatment step.

The nitrification tank and the denitrification tank may be separate tanks, or may be the same tank. When the nitrification tank and the denitrification tank are the same, the nitrification carrier carrying the nitrifying bacterium and the microbial carrier carrying the denitrifying bacterium may be placed in one tank, and the nitrification carrier and the microbial carrier may be separated by a fibrous separator, a filter paper, or the like. The nitrification tank, the nitrifying bacterium, the nitrification carrier, the fibrous separator, the filter paper, and the like can be appropriately selected and used from known ones.

As described above, with the microbial carrier and water treatment method according to the embodiment of the present invention, hydrolysis of a biodegradable resin constituting a microbial carrier proceeds favorably by the activity of the microorganism fixed on the microbial carrier, and the biofilm is favorably formed on the microbial carrier, so that the denitrification efficiency is stabilized and improved.

In addition, with the microbial carrier and water treatment method according to the embodiment of the present invention, since iron is efficiently replenished to the microorganism including the denitrifying bacterium, which is fixed on the iron-containing carrier, the iron deficiency is suppressed and the denitrification is improved.

The technical scope of the present invention is not limited to the above-described embodiments, and various modifications can be made within the scope of the present invention.

[Examples]

Hereinafter, the present invention will be described in more detail with reference to Examples, but the present invention is not limited to aspects shown in the following examples.

<Examples 1 to 24 and Comparative Examples 1 to 6>

(Production of microbial carrier)

Carriers A1 to A14, carrier a1, carrier a2, carriers B 1 to B4, and carrier c were produced by the following methods.

"Production of carrier A1"

200 g of pellet-like poly(butylene succinate/adipate) (PBSA; manufactured by PTT MCC Biochem Co., Ltd.; short diameter: approximately 3 mm; long diameter: approximately 6 mm) (hereinafter, described as PBSA) was put into a heat-resistant PTFE universal container 120φ (manufactured by Flonchemical Co., Ltd.) (hereinafter, described as a PTFE dish). Furthermore, powdery iron (III) oxide red (manufactured by Hayashi Pure Chemical Ind., Ltd.) and powdery tricalcium phosphate (manufactured by KANTO KAGAKU) were added thereto in an amount shown in the column of "Addition amount of iron compound with respect to 200 g of PBSA (g)" and "Addition amount of phosphorus compound with respect to 200 g of PBSA (g)" in Table 1 below. The total amount of added iron (III) oxide and tricalcium phosphate was mixed with the PBSA by stirring and mixing using a PTFE rod (diameter: 12 mm, length: 300 mm) (hereinafter, described as a PTFE rod). Thereafter, the PBSA in the PTFE dish was heated in a thermostatic bath at 125°C for 30 minutes, and the PBSA was melted. Thereafter, the melted PBSA was kneaded with the iron (III) oxide using the PTEF rod. Thereafter, the PBSA in the PTFE dish was heated again at 125°C for 30 minutes to melt the PBSA. Thereafter, the entire amount of the melted PBSA was transferred onto a heat-resistant PTFE plate (50 mm × 50 mm), and pressed with the PTEF rod to be molded into a plate having a thickness of 3 mm and a size of approximately 3000 mm × 300 mm. Thereafter, the plate was cut into a chip having a side of 10 mm, a side of 10 mm, and a thickness of 3 mm using scissors to produce carrier A1.

"Production of carriers A3 to A5, carrier a1, and carriers B2 to B4"

Carriers A3 to A5, carrier a1, and carriers B2 to B4 were produced by the same method as carrier A1, except that the addition amounts of iron (III) oxide and tricalcium phosphate were changed as shown in the column of "Addition amount of iron compound with respect to 200 g of PBSA (g)" of each carrier in Table 1.

"Production of carrier A2"

Carrier A2 was produced by the same method as carrier A1, except that iron (II, III) oxide was added instead of iron (III) oxide.

"Production of carrier A6"

Carrier A6 was produced by the same method as carrier A1, except that ferrous sulfate (II) heptahydrate was added instead of iron (III) oxide.

"Production of carriers A7, A9 to A13, and carrier a2"

Carriers A7, A9 to A13, and a carrier a2 were produced by the same method as carrier A1, except that tricalcium phosphate was not added.

"Production of carrier A8"

Carrier A8 was produced by the same method as carrier A1, except that iron (II, III) oxide was added instead of iron oxide (III), and tricalcium phosphate was not added.

"Production of carrier A14"

Carrier A14 was produced by the same method as carrier A1, except that ferrous sulfate (II) heptahydrate was added instead of iron oxide (III), and tricalcium phosphate was not added.

"Production of carrier B 1"

Carrier B 1 was produced by the same method as carrier A1, except that iron (III) oxide was not added.

"Production of carrier c"

Carrier c was produced by the same method as carrier A1, except that iron (III) oxide and tricalcium phosphate were not added.

[Table 1]

| Carrier | Biodegradable resin | Iron compound | Phosphorus compound | Iron content C(Fe) | Phosphorus content C(P) | Molar ratio X 'phosphorus/iron) | Molar ratio Y (iron/phosphorus) | Addition amount of iron compound with respect to 200 g of PBSA | Addition amount of phosphorus compound with respect to 200 g of PBSA |
|---|---|---|---|---|---|---|---|---|---|
| | | | | % by mass | % by mass | mol/mol | mol/mol | g | g |
| Carrier A1 | PBSA | $Fe_2O_3$ | $Ca_3(PO_4)_2$ | $4.0 \times 10^{-2}$ | $1.0 \times 10^{-3}$ | 0.045 | 22.186 | 0.114 | 0.010 |
| Carrier A2 | PBSA | $Fe_3O_4$ | $Ca_3(PO_4)_2$ | $4.0 \times 10^{-2}$ | $1.0 \times 10^{-3}$ | 0.045 | 22.186 | 0.111 | 0.010 |
| Carrier A3 | PBSA | $Fe_2O_3$ | $Ca_3(PO_4)_2$ | $4.0 \times 10^{-2}$ | $2.0 \times 10^{-3}$ | 0.090 | 11.093 | 0.114 | 0.020 |
| Carrier A4 | PBSA | $Fe_2O_3$ | $Ca_3(PO_4)_2$ | $4.0 \times 10^{-2}$ | $3.0 \times 10^{-3}$ | 0.135 | 7.395 | 0.114 | 0.030 |
| Carrier A5 | PBSA | $Fe_2O_3$ | $Ca_3(PO_4)_2$ | $4.0 \times 10^{-2}$ | $1.0 \times 10^{-2}$ | 0.451 | 2.219 | 0.114 | 0.100 |
| Carrier A6 | PBSA | $FeSO_4 \cdot 7H_2O$ | $Ca_3(PO_4)_2$ | $4.0 \times 10^{-2}$ | $1.0 \times 10^{-3}$ | 0.045 | 22.186 | 0.398 | 0.010 |
| Carrier A7 | PBSA | $Fe_2O_3$ | - | $5.0 \times 10^{-3}$ | 0.0 | 0.000 | - | 0.014 | 0.000 |
| Carrier A8 | PBSA | $Fe_3O_4$ | - | $4.0 \times 10^{-2}$ | 0.0 | 0.000 | - | 0.111 | 0.000 |
| Carrier A9 | PBSA | $Fe_2O_3$ | - | $1.0 \times 10^{-1}$ | 0.0 | 0.000 | - | 0.286 | 0.000 |
| Carrier A10 | PBSA | $Fe_2O_3$ | - | $5.0 \times 10^{-1}$ | 0.0 | 0.000 | - | 1.430 | 0.000 |
| Carrier A11 | PBSA | $Fe_2O_3$ | - | $2.0 \times 10^{-3}$ | 0.0 | 0.000 | - | 0.006 | 0.000 |
| Carrier A12 | PBSA | $Fe_2O_3$ | - | 1.0 | 0.0 | 0.000 | - | 2.859 | 0.000 |

| Carrier | Biodegradable resin | Iron compound | Phosphorus compound | Iron content C(Fe) | Phosphorus content C(P) | Molar ratio X 'phosphorus/iron) | Molar ratio Y (iron/phosphorus) | Addition amount of iron compound with respect to 200 g of PBSA | Addition amount of phosphorus compound with respect to 200 g of PBSA |
|---|---|---|---|---|---|---|---|---|---|
| | | | | % by mass | % by mass | mol/mol | mol/mol | g | g |
| Carrier A13 | PBSA | $Fe_2O_3$ | - | $1.0 \times 10^{-3}$ | 0.0 | 0.000 | - | 0.003 | 0.000 |
| Carrier A14 | PBSA | $FeSO_4 \cdot 7H_2O$ | - | $4.0 \times 10^{-2}$ | 0.0 | 0.000 | - | 0.398 | 0.000 |
| Carrier a1 | PBSA | $Fe_2O_3$ | $Ca_3(PO_4)_2$ | $4.0 \times 10^{-2}$ | $1.5 \times 10^{-2}$ | 0.676 | 1.479 | 0.114 | 0.150 |
| Carrier a2 | PBSA | $Fe_2O_3$ | - | 2.0 | 0.0 | 0.000 | - | 5.719 | 0.000 |
| Carrier B1 | PBSA | - | $Ca_3(PO_4)_2$ | 0.0 | $2.0 \times 10^{-2}$ | - | 0.000 | 0.000 | 0200 |
| Carrier B2 | PBSA | $Fe_2O_3$ | $Ca_3(PO_4)_2$ | $1.0 \times 10^{-3}$ | $2.0 \times 10^{-2}$ | 36.059 | 0.028 | 0.003 | 0200 |
| Carrier B3 | PBSA | $Fe_2O_3$ | $Ca_3(PO_4)_2$ | $3.0 \times 10^{-3}$ | $2.0 \times 10^{-2}$ | 12.020 | 0.083 | 0.009 | 0200 |
| Carrier B4 | PBSA | $Fe_2O_3$ | $Ca_3(PO_4)_2$ | $5.0 \times 10^{-3}$ | $2.0 \times 10^{-2}$ | 7.212 | 0.139 | 0.014 | 0200 |
| Carrier c | PBSA | - | - | 0.0 | 0.0 | - | - | 0.000 | 0.000 |

(Adjustment of acclimated sludge)

Suspension water was collected from a denitrification tank of purification treatment equipment at an aquaculture farm, and was concentrated by centrifugal separation at 3,000 rpm for 5 minutes to produce a sludge having an amount of mixed liquor suspended solids (MLSS) of 3,600 mg/L.

(Denitrification test)

Simulated wastewater was prepared by dissolving sodium nitrate ($NaNO_3$), dipotassium hydrogenphosphate ($K_2HPO_4$), magnesium sulfate heptahydrate ($MgSO_4 \cdot 7H_2O$), ethylenediaminetetraacetic acid disodium salt (EDTA), and calcium chloride dihydrate ($CaCl_2 \cdot H_2O$) in pure water such that concentrations of nitric acid nitrogen, phosphorus, magnesium ions, sodium ions, and calcium ions were respectively 100 mg-N/L, 5 mg-P/L, 8.8 mg-Mg/L, 1.03 mg/Na/L, and 0.57 mg-Ca/L.

Next, a denitrification test was performed using the circulation-type denitrification treatment system shown in FIG. 1.

First, 200 g of the microbial carriers of Examples 1 to 24 and Comparative Examples 1 to 6, which were produced by combining the microbial carriers at mixing ratios shown in the column of "Microbial carrier" in Table 2 below in the denitrification column 105 having an inner diameter of 3.5 mm, a length of 450 mm, and an effective volume of 300 mL.

Next, 20 mL of the seed sludge was mixed with 2,000 mL of the water in the water storage tank 101 having a capacity of 2.2 L, and the water was fed at a water flow rate of 10 mL/min by the pump 102 to circulate the water.

As shown in FIG. 1, in the denitrification test, the water in the water storage tank 101 was circulated in the denitrification treatment system by being supplied to the inside of the denitrification column 105 from a supply port at the lower part of the denitrification column through the supply tube 103, being discharged from a return port at the upper part of the denitrification column, and then being returned to the water storage tank 101 through the return tube 106.

A nitric acid nitrogen concentration C (mg/L) of the water in the water storage tank 101 on the 10th day from the start of the test was measured with a compact nitrate ion meter (manufactured by HORIBA Advanced Techno, Co., Ltd.; "LAQUAtwin NO3-11"). Table 2 below shows the nitric acid nitrogen concentration after the denitrification test.

[Table 2]

| | | Microbial carrier | | | | Denitrification test result |
|---|---|---|---|---|---|---|
| | | Carrier A | Carrier B | Other carriers | Mixing ratio carrier A/carrier B | Nitric acid nitrogen in water to be treated after denitrification test |
| | | | | | | mg/l |
| | Example 1 | Carrier A1 | Carrier B 1 | - | 50/50 | 2 |
| | Example 2 | Carrier A7 | Carrier B 1 | - | 50/50 | 2 |
| | Example 3 | Carrier A7 | Carrier B2 | - | 50/50 | 4 |
| | Example 4 | Carrier A7 | Carrier B3 | - | 50/50 | 7 |
| | Example 5 | Carrier A7 | Carrier B4 | - | 50/50 | 14 |
| | Example 6 | Carrier A7 | Carrier B 1 | - | 10/90 | 8 |
| | Example 7 | Carrier A7 | Carrier B1 | - | 90/10 | 7 |
| | Example 8 | Carrier A7 | - | - | 100/0 | 12 |
| | Example 9 | Carrier A2 | - | - | 100/0 | 13 |
| | Example 10 | Carrier A7 | - | - | 100/0 | 14 |
| | Example 11 | Carrier A8 | - | - | 100/0 | 14 |
| | Example 12 | Carrier A9 | - | - | 100/0 | 14 |
| | Example 13 | Carrier A10 | - | - | 100/0 | 19 |
| | Example 14 | Carrier A11 | - | - | 100/0 | 18 |
| | Example 15 | Carrier A12 | - | - | 100/0 | 24 |

(continued)

| | Microbial carrier | | | | Denitrification test result |
| --- | --- | --- | --- | --- | --- |
| | Carrier A | Carrier B | Other carriers | Mixing ratio carrier A/carrier B | Nitric acid nitrogen in water to be treated after denitrification test |
| | | | | | mg/l |
| Example 16 | Carrier A13 | - | - | 100/0 | 25 |
| Example 17 | Carrier A3 | Carrier B1 | - | 50/50 | 32 |
| Example 18 | Carrier A3 | - | - | 100/0 | 35 |
| Example 19 | Carrier A5 | Carrier B1 | - | 50/50 | 37 |
| Example 20 | Carrier A4 | - | - | 100/0 | 42 |
| Example 21 | Carrier A5 | - | - | 100/0 | 48 |
| Example 22 | Carrier A14 | - | - | 100/0 | 56 |
| Example 23 | Carrier A6 | Carrier B1 | - | 50/50 | 58 |
| Example 24 | Carrier A6 | - | - | 100/0 | 58 |
| Comparative Example 1 | Carrier a1 | - | - | 100/0 | 96 |
| Comparative Example 2 | - | - | Carrier c | (Carrier c = 100) | 99 |
| Comparative Example 3 | Carrier a1 | Carrier B1 | - | 50/50 | 96 |
| Comparative Example 4 | - | Carrier B1 | Carrier c | (Carrier B/c = 50/50) | 99 |
| Comparative Example 5 | - | Carrier B1 | - | 0/100 | 99 |
| Comparative Example 6 | Carrier a2 | - | - | 100/0 | 76 |

As shown in Tables 1 and 2, when the value of the molar ratio (phosphorus/iron) X of the phosphorus to the iron, comprised in carrier A, was 0.5 or less (Examples 1 to 24), the value of nitric acid nitrogen of the water after the denitrification test was lower than that in Comparative Examples 1 and 3 in which the value was more than 0.5. A high denitrification efficiency was obtained.

In Examples in which the iron content of the iron-containing carrier was less than 2.0% by mass with respect to the mass of the iron-containing carrier, it was found that the value of nitric acid nitrogen in the water after the denitrification test was lower than that in Comparative Example 6 in which the iron content of the iron-containing carrier was 2.0% by mass with respect to the mass of the iron-containing carrier, and a high denitrification efficiency was exhibited.

In addition, in Examples 1 to 21 in which the iron compound comprised trivalent iron, a higher denitrification efficiency was obtained.

Furthermore, when the value of the molar ratio (phosphorus/iron) X of the phosphorus to the iron, comprised in carrier A, was 0.05 or less (Examples 1 to 16), particularly high denitrification efficiency was obtained.

In Comparative Examples 2, 4, and 5, a microbial carrier not containing carrier A was used. In this case, it was considered that the denitrification was stagnant because the iron required for the denitrification was not replenished in the microorganism, and the nitric acid nitrogen in the water did not decrease.

From the above, it was found that, by using a microbial carrier which comprised an iron-containing carrier comprising a biodegradable resin and iron having a molar ratio (phosphorus/iron) X of phosphorus to iron of 0.5 or less, a high denitrification efficiency could be achieved.

[Industrial Applicability]

According to the microbial carrier and water treatment method of the present invention, denitrification efficiency is stabilized and improved.

[Reference Signs List]

101: Water storage tank
102: Pump
103: Supply tube
104: Microbial carrier
105: Denitrification column
106: Return tube

**Claims**

1. A microbial carrier which carries a microorganism having denitrification ability, the microbial carrier comprising:

   an iron-containing carrier which comprises a biodegradable resin and iron,
   wherein, when the iron-containing carrier comprises phosphorus, a molar ratio (phosphorus/iron) X of the phosphorus to the iron in the iron-containing carrier is $0 < X \leq 0.5$,
   when the iron-containing carrier comprises no phosphorus, the molar ratio (phosphorus/iron) X is X = 0, and
   an iron content of the iron-containing carrier is $1.0 \times 10^{-4}$% by mass or more and less than 2.0% by mass with respect to a mass of the iron-containing carrier.

2. The microbial carrier according to Claim 1, further comprising:

   a phosphorus-containing carrier which comprises a biodegradable resin and phosphorus,
   wherein, when the phosphorus-containing carrier comprises iron, a molar ratio (iron/phosphorus) Y of iron to the phosphorus in the phosphorus-containing carrier is $0 < Y \leq 0.1$, and
   when the phosphorus-containing carrier comprises no iron, the molar ratio (iron/phosphorus) Y is Y = 0.

3. The microbial carrier according to Claim 1,
   wherein the iron-containing carrier comprises trivalent iron.

4. The microbial carrier according to Claim 1,
   wherein the biodegradable resin is a biodegradable polyester.

5. The microbial carrier according to Claim 4,
   wherein the biodegradable polyester includes two or more types of constitutional units derived from a dicarboxylic acid.

6. The microbial carrier according to any one of Claims 1 to 5,
   wherein the microbial carrier is a microbial carrier which further carries a microorganism having an ability to decompose the biodegradable resin.

7. A water treatment method using a microbial carrier which carries a microorganism having denitrification ability,

   wherein the microbial carrier comprises an iron-containing carrier comprising a biodegradable resin and iron,
   when the iron-containing carrier comprises phosphorus, a molar ratio (phosphorus/iron) X of the phosphorus to the iron in the iron-containing carrier is $0 < X \leq 0.5$,
   when the iron-containing carrier comprises no phosphorus, the molar ratio (phosphorus/iron) X is X = 0, and
   an iron content of the iron-containing carrier is $1.0 \times 10^{-4}$% by mass or more and less than 2.0% by mass with respect to a mass of the iron-containing carrier.

8. The water treatment method according to Claim 7,

   wherein the microbial carrier further comprises a phosphorus-containing carrier comprising a biodegradable resin and phosphorus,
   when the phosphorus-containing carrier comprises iron, a molar ratio (iron/phosphorus) Y of iron to the phosphorus in the phosphorus-containing carrier is $0 < Y \leq 0.1$, and

when the phosphorus-containing carrier comprises no iron, the molar ratio (iron/phosphorus) Y is $Y = 0$.

FIG. 1

## INTERNATIONAL SEARCH REPORT

| | |
|---|---|
| International application No. | |
| | **PCT/JP2023/010639** |

**A. CLASSIFICATION OF SUBJECT MATTER**

***C12N 11/096***(2020.01)i; ***C02F 3/34***(2023.01)i
FI: C02F3/34 101D; C12N11/096

According to International Patent Classification (IPC) or to both national classification and IPC

**B. FIELDS SEARCHED**

Minimum documentation searched (classification system followed by classification symbols)

C12N11/00-18; C02F3/00-34; C02F11/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2007-125460 A (HITACHI PLANT TECHNOLOGIES, LTD.) 24 May 2007 (2007-05-24) claims, paragraphs [0032]-[0072], examples | 1, 3, 7 |
| Y | claims, paragraphs [0032]-[0072], examples | 4-6 |
| X | CN 111072132 A (BEIJING ENFI ENVIRONMENTAL PROTECTION CO., LTD.) 28 April 2020 (2020-04-28) claims, paragraphs [0017]-[0018], [0021]-[0023] | 1, 7 |
| X | JP 10-296284 A (TAKEDA CHEM. IND., LTD.) 10 November 1998 (1998-11-10) claims, paragraphs [0005]-[0012] | 1-3, 7-8 |
| Y | claims, paragraphs [0005]-[0012] | 4-6 |
| Y | JP 2004-136182 A (DAINIPPON PLASTICS CO., LTD.) 13 May 2004 (2004-05-13) paragraphs [0001]-[0002], [0009]-[0010], [0016], [0025] | 4-6 |
| A | JP 2004-8923 A (TAIHO IND. CO., LTD.) 15 January 2004 (2004-01-15) | 1-8 |
| A | JP 10-327850 A (RES. DEV. CORP. OF JAPAN) 15 December 1998 (1998-12-15) | 1-8 |

☑ Further documents are listed in the continuation of Box C.   ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **22 May 2023** | **06 June 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2023/010639**

| C. | DOCUMENTS CONSIDERED TO BE RELEVANT | |
|---|---|---|

| Category* | Citation of document, with indication, where appropriate, of the relevant passages | Relevant to claim No. |
|---|---|---|
| A | JP 2021-186805 A (HUBEI HUINONG BIOLOGICAL TECHNOLOGY CO., LTD.) 13 December 2021 (2021-12-13) | 1-8 |
| A | CN 109294948 A (BEIJING JIAOTONG UNIVERSITY) 01 February 2019 (2019-02-01) | 1-8 |

Form PCT/ISA/210 (second sheet) (January 2015)

INTERNATIONAL SEARCH REPORT
Information on patent family members

International application No.

**PCT/JP2023/010639**

| Patent document cited in search report | | | Publication date (day/month/year) | Patent family member(s) | | | Publication date (day/month/year) |
|---|---|---|---|---|---|---|---|
| JP | 2007-125460 | A | 24 May 2007 | US paragraphs [0038]-[0069], examples, claims EP | 2007/0119776 1780271 | A1 A1 | |
| CN | 111072132 | A | 28 April 2020 | (Family: none) | | | |
| JP | 10-296284 | A | 10 November 1998 | (Family: none) | | | |
| JP | 2004-136182 | A | 13 May 2004 | (Family: none) | | | |
| JP | 2004-8923 | A | 15 January 2004 | (Family: none) | | | |
| JP | 10-327850 | A | 15 December 1998 | US EP CN | 2001/0024828 881287 1203273 | A1 A2 A | |
| JP | 2021-186805 | A | 13 December 2021 | US GB EP | 2021/0380449 2599194 3919447 | A1 A A2 | |
| CN | 109294948 | A | 01 February 2019 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022059131 A
- JP 2004175848 A
- JP 2004008923 A